(19)

Europäisches
Patentamt
European
Patent Office
Office européen
des brevets

(11) **EP 4 548 836 A1**

(12) **EUROPEAN PATENT APPLICATION**
published in accordance with Art. 153(4) EPC

(43) Date of publication:
**07.05.2025 Bulletin 2025/19**

(21) Application number: **23830827.4**

(22) Date of filing: **01.05.2023**

(51) International Patent Classification (IPC):
*A61B 5/01* (2006.01)          *G01K 7/42* (2006.01)
*G01K 13/20* (2021.01)

(52) Cooperative Patent Classification (CPC):
**A61B 5/01; G01K 7/42; G01K 13/20**

(86) International application number:
**PCT/JP2023/017069**

(87) International publication number:
**WO 2024/004375 (04.01.2024 Gazette 2024/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.06.2022 JP 2022105509**

(71) Applicant: **Public University Corporation Suwa
University of
Science Foundation
Chino-shi Nagano, 391-0292 (JP)**

(72) Inventor: **HASHIMOTO Nobuaki
Chino-shi Nagano 391-0292 (JP)**

(74) Representative: **Liesegang, Eva
Boehmert & Boehmert
Anwaltspartnerschaft mbB
Pettenkoferstrasse 22
80336 München (DE)**

(54) **PROBE FOR DEEP TEMPERATURE MEASUREMENT AND DEEP THERMOMETER**

(57)    A deep part temperature measuring probe 1 is a deep part temperature measuring probe used at a time of measuring a temperature of a deep part of a subject 9. Such a deep part temperature measuring probe 1 includes: a plate-like board 10; a pair of first region temperature sensors 21,22 that is mounted on the board 10 in a first region 11 of the board 10 in a state where the pair of first region temperature sensors 21,22 face each other while sandwiching the board 10 therebetween; and a pair of second region temperature sensors 23, 24 that is a pair of temperature sensors 23, 24 mounted on the board 10 in a second region 12 of the board 10 in a state where the pair of second region temperature sensors 23, 24 face each other while sandwiching the board 10 therebetween. A through hole 15 that penetrates the board 10 between a front surface 10a and a back surface 10b of the board 10 is formed just below the first region temperature sensors 21, 22, and the pair of first region temperature sensors 21, 22 are connected to each other through the through hole 15. According to the deep part temperature measuring probe 1, even in a case where the board that forms the probe is made thin, a temperature of a subject can be measured with high accuracy.

FIG. 1

**Description**

Technical Field

The present invention relates to a deep part temperature measuring probe and a deep part thermometer, mainly a deep part temperature measuring probe and a deep part thermometer for measuring a deep part temperature (core body temperature) of a human body.

Background Art

Conventionally, as a non-invasion method, there has been studied a dual-heat flow method that measures a temperature of a deep part of a human body. The dual-heat flow method is a method that obtains a deep part temperature TB without using a heat resistance value of a biological skin that is an unknown number by solving a simultaneous equation relating to heat flows by measuring the heat flows that flow through two different heat insulating materials (for example, see non-patent literature 1).

In non-patent literature 1 and patent literature 1, a deep part temperature measuring probe (hereinafter, simply referred to as "probe") that measures a deep part temperature TB using a dual-heat flow method is disclosed. To constitute a probe used for a dual-heat flow method, it is essential to generate a difference between heat resistance values of two heat flow paths (between a heat resistance value of a first heat flow path and a heat resistance value of a second heat flow path). Accordingly, also in these literatures, probes formed in accordance with such a design concept have been disclosed.

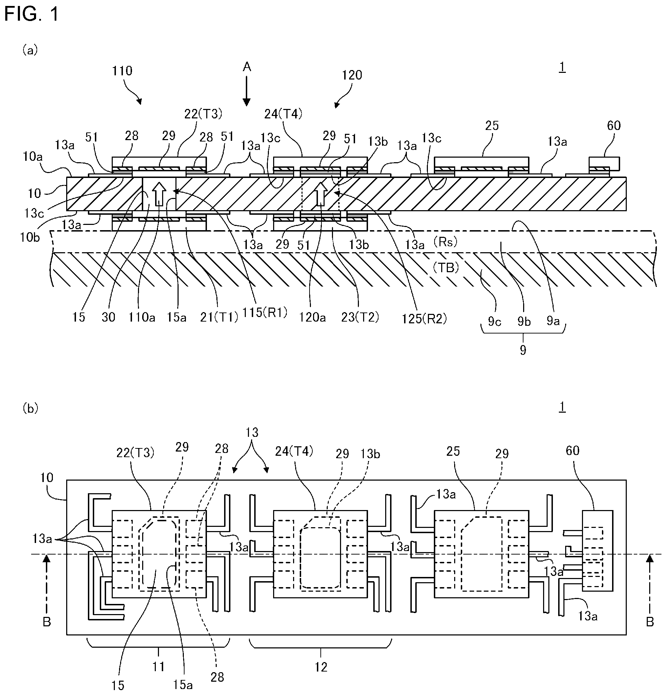

The deep part temperature measuring probe described in patent literature 1 is configured such that a difference in a heat resistance ratio is generated between a first region of a board and a second region of the board by making an occupation ratio and/or dispersion of a conductive pattern in the first region of the board and an occupation ratio and/or dispersion of the conductive pattern in the second region of the board differ from each other. For example, in an example illustrated in Fig. 1, in the first heat flow path, the conductive pattern is disposed between layers of the board thus lowering an entire heat resistance value R. On the other hand, in the second heat flow path, such an interlayer conductive pattern is not disposed so that the second flow path is formed using only a board material itself. Accordingly, as a whole, resistance values of both heat flow paths are made different from each other.

Citation List

Non-Patent Literature

[Non-Patent Literature 1] Issei Yanai, "Development of a non-invasive deep body thermometer and its experimental evaluation", master course thesis of Information Science Research Course of Nara institute of Science and Technology, NAIST Digital Library 2014

Patent Literature

[Patent Literature 1] WO 2019/167707

Summary of Invention

Technical Problem

Recently, a temperature often becomes extremely high during the summer season. Accordingly, it is estimated that the number of attempts will be increased for monitoring a deep part temperature TB of a living body (particularly, a deep part temperature of a human body) by wearing a deep part temperature measuring probe on the living body from a viewpoint of preventing a heat stroke or the like. As an attribute that a wearable probe is expected to possess, it is important for the probe to be "of a thin type" from a viewpoint of enhancing a wearing comfort by lowering feeling of resistance in wearing.

However, when a thickness of a board that constitutes the deep part temperature measuring probe becomes thin, a size of the probe in the thickness direction is decreased and hence, a tendency that lowering of a heat resistance value in the thickness direction progresses. In this case, according to the technique of the deep part temperature measuring probe described in patent literature 1, even when the conductive pattern is inserted between layers for decreasing a heat resistance value of the first region, a heat resistance value of the second region is also decreased due to the thinning of the board and hence, it is difficult to provide an apparent difference in a heat resistance value between two regions. Accordingly, even in a case where a thin wearable deep part temperature measuring probe is formed by the technique described in patent literature 1, it is difficult to maintain high accuracy in measurement and estimation (simply referred to as "measurement") of the deep part temperature.

Further, in the deep part temperature measuring probe described in patent literature 1, the smaller the size of the board, the more difficult a built-in operation of a conductive pattern in the board becomes and hence, the realization and the practical use of such a deep part temperature measuring probe cannot be achieved.

The present invention has been made in view of such circumstances, and it is an object of the present invention to provide a deep part temperature measuring probe that can perform the measurement of a temperature of a deep part of a subject with high accuracy even in a case where a board that constitutes the probe is made thin. It is another object of the present invention to provide a deep part thermometer that includes a deep part temperature measuring probe.

Solution to Problem

According to one aspect of the present invention, there is provided a deep part temperature measuring probe that is used at a time of measuring a temperature of a deep part of a subject. Such a deep part temperature measuring probe includes: a plate-like board; a pair of first region temperature sensors" that is a pair of temperature sensors mounted on the board in a first region of the board in a state where the pair of first region temperature sensors face each other while sandwiching the board therebetween; and a pair of second region temperature sensors" that is a pair of temperature sensors mounted on the board in a second region of the board in a state where the pair of second region temperature sensors face each other while sandwiching the board therebetween. A through hole that penetrates the board between a front surface and a back surface of the board is formed just below the first region temperature sensor, and the pair of first region temperature sensors are connected to each other through the through hole.

According to another aspect of the present invention, there is provided a deep part temperature measuring probe that is used at a time of measuring a temperature of a deep part of a subject in the same manner.

Such a deep part temperature measuring probe includes: a plate-like board; a first heat flow measuring system that includes "a pair of first region temperature sensors" that is a pair of temperature sensors mounted on the board in a first region of the board in a state where the pair of first region temperature sensors faces each other while sandwiching the board therebetween, and a first heat flow path formed in the board in the first region, and measures a first heat flow that flows out from a subject. The deep part temperature measuring probe also includes a second heat flow measuring system that includes "a pair of second region temperature sensors" that is a pair of temperature sensors mounted on the board in a second region of the board in a state where the pair of second region temperature sensors faces each other while sandwiching the board therebetween; and a second heat flow path formed in the board in the second region, and measures a second heat flow that flows out from the subject. Further, a through hole that penetrates the board between a front surface and a back surface of the board is formed just below the first region temperature sensor, and the first heat flow path is formed of an air layer disposed in the through hole.

Still another aspect of the present invention, there is provided a deep part thermometer that includes: the deep part temperature measuring probe described above; and a deep part temperature estimating unit that estimates a deep part temperature using respective temperatures measured by the pair of first region temperature sensors and the pair of second region temperature sensors of the deep part temperature measuring probe.

Advantageous effects of Invention

According to the present invention, it is possible to provide the deep part temperature measuring probe capable of measuring a temperature of a deep part of the subject with high accuracy even if the board that constitutes the probe is made thin.

Brief Description of Drawings

Fig. 1 is a view illustrating a deep part temperature measuring probe 1 according to an embodiment 1.
Fig. 2 is a view illustrating a constitutional element of the deep part temperature measuring probe 1 according to the embodiment 1.
Fig. 3 is a block diagram illustrating one example of the hardware configuration of a deep part thermometer 500 according to the embodiment 1.
Fig. 4 is a view illustrating a thermal equivalent circuit of the deep part temperature measuring probe 1 according to the embodiment 1.
Fig. 5 is a schematic cross-sectional view illustrating an experimental system of the deep part temperature measuring probe 1 according to the embodiment 1.

Fig. 6 is a cross-sectional view illustrating a deep part temperature measuring probe 2 according to an embodiment 2.
Fig. 7 is a cross-sectional view illustrating a deep part temperature measuring probe 3 according to an embodiment 3.
Fig. 8 is a view illustrating a deep part temperature measuring probe 4 according to an embodiment 4.
Fig. 9 is a cross-sectional view illustrating a deep part temperature measuring probe 5 according to a modification 1.
Fig. 10 is constituted of cross-sectional views illustrating deep part temperature measuring probes 6, 7 according to a modification 2 and a modification 3.
Fig. 11 is constituted of views illustrating deep part temperature measuring probes 4, 4' according to a modification 4.

Description of Embodiments

Hereinafter, the description is made with respect to a deep part temperature measuring probe and a deep part thermometer according to the present invention with reference to drawings. With respect to the substantially same symbols in the respective drawings, contents that are already described with reference to these symbols can be used in the description of other drawings and hence, the description of these contents is omitted.

[Embodiment 1]1. Configuration of deep part temperature measuring probe 1 according to embodiment 1

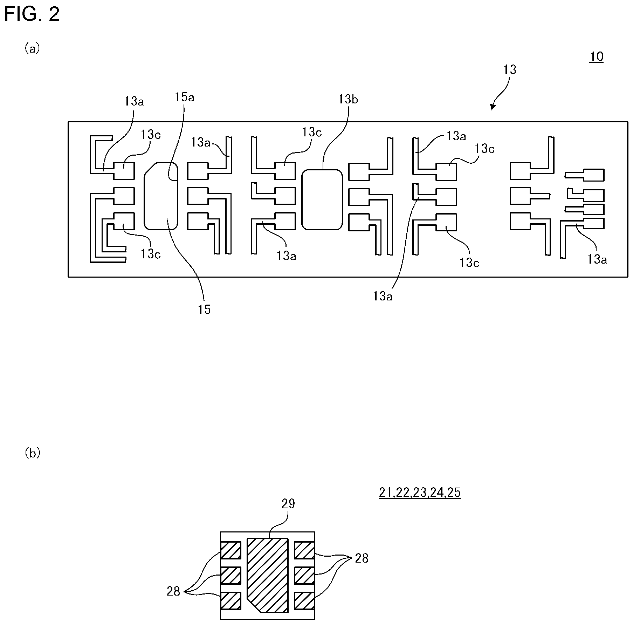

Fig. 1 is a view illustrating a deep part temperature measuring probe 1 according to an embodiment 1. Fig. 1(a) is a cross-sectional view of the deep part temperature measuring probe 1 taken along a line B-B in Fig. 1(b). Fig. 1(b) is a plan view of the deep part temperature measuring probe 1 when the deep part temperature measuring probe 1 is viewed along an arrow A in Fig. 1(a). Fig. 2 is a view illustrating constitutional elements of the deep part temperature measuring probe 1 according to the embodiment 1.

(1) Overall configuration of deep part temperature measuring probe 1

As illustrated in Fig. 1, the deep part temperature measuring probe 1 is a probe that is used at the time of measuring a temperature of a subject deep part 9c that is a deep part of the subject 9. The deep part temperature measuring probe 1 measures a temperature (deep part temperature TB) of the subject deep part 9c by bringing a back surface side of the deep part temperature measuring probe 1 into contact with a subject surface 9a directly or indirectly. In the drawing, symbol RS expresses a thermal resistance value of a subject intermediate portion 9b whose temperature cannot be directly measured.

As a typical subject 9, a living body such as a human or a beast is named. However, in this embodiment, the description is made hereinafter by estimating a human as the subject 9.

The deep part temperature measuring probe 1 includes a board 10, temperature sensors 21, 22, 23, 24. In this specification, there may be a case where the temperature sensor is simply referred to as "a sensor".

(2) Board 10

Fig. 2 (a) is a plan view illustrating the board 10. Fig. 2 (a) illustrates a mode when a surface of the bare board on which the temperature sensors 21, 22, 23, 24, 25 are not mounted on the board is viewed.

As illustrated in Fig. 2 (a), the board 10 is a printed circuit board on which a wiring pattern 13 is formed. As the board 10, for example, glass epoxy board can be adopted. The board 10 according to this embodiment is formed of a so-called double sided printed wiring board on which the wiring pattern 13 is formed on both surfaces consisting of a front surface 10a and a back surface 10b. The wiring pattern 13 (the wiring pattern in a broad meaning of the term) includes terminals 13c, wiring patterns 13a in the narrow meaning of the term connected between the terminals 13c, a land 13b in the narrow meaning of the term formed in a pad shape and the like. In the drawing, with respect to the wiring pattern 13a in the narrow meaning of the term, the description of the intermediate connection is omitted.

The board 10 has the front surface 10a and the back surface 10b, and is formed in a plate-like shape, for example. In this embodiment, the definitions of the front surface 10a and the back surface 10b are made for the sake of convenience. A surface that faces an external field (an atmosphere 8 side) is assumed as the front surface 10a, and a surface on a side that is brought into contact with the subject 9 is assumed as the back surface 10b.

The board 10 has a function of a base portion on which the temperature sensors 21, 22, 23, 24 are mounted and/or a portion (or an entirety) of the board constitutes a flow path of heat (heat flow path) where heat flows from a side of the back surface 10b to a side of the front surface 10a (see Fig. 1 also).

(3) Temperature sensors 21, 22, 23, 24

The temperature sensors 21, 22, 23, 24 measure temperatures at contact portions (nodes), and output the output signals corresponding to measured temperatures. Each of the temperature sensors 21, 22, 23, 24 is formed of a discrete device such as, for example, a thermocouple, a platinum temperature measurement register, a thermistor, or a device that is formed into an IC (integrated circuit) and outputs an output signal digitally.

As the temperature sensors 21, 22, 23, 24 of this embodiment, a temperature sensor formed as an IC is used. For example, as illustrated in the drawing, the temperature sensors 21, 22, 23, 24 may be realized by a so-called small outline non-leaded package (SON package).

Fig. 2 (b) is a bottom surface view illustrating a bottom surface of the SON package in a case where the temperature sensors 21, 22, 23, 24, 25 are realized by the SON package. Each of the temperature sensors 21, 22, 23, 24, 25 incorporates a semiconductor chip having a function of sensing a temperature and converting the temperature into an electric signal (not illustrated in the drawing), and external connecting terminals 28 that are electrically connected to the semiconductor chips are exposed on the bottom surface. Further, each of the temperature sensors 21, 22, 23, 24, 25 has a thermal pad 29 that improves thermal coupling between a contact portion to be measured (specifically, a node of a heat flow path) and the semiconductor chip incorporated in the temperature sensor on a bottom surface of each temperature sensor.

Returning to Fig. 1, the mounting of temperature sensors on the board is described.

The temperature sensors 21, 22 are mounted on the board 10 as a pair in a state where the temperature sensors 21, 22 opposedly face each other while sandwiching the board 10 therebetween in a first region 11 of the board 10. The temperature sensor 21 is mounted on the back surface 10b of the board 10, the temperature sensor 22 is mounted on the front surface 10a of the board 10. In a same manner, the temperature sensors 23, 24 are mounted on the board 10 as a pair in a state where the temperature sensors 23, 24 opposedly face each other while sandwiching the board 10 in a second region 12 of the board 10. The temperature sensor 23 is mounted on the back surface 10b of the board 10, and the temperature sensor 24 is mounted on the front surface 10a of the board 10.

In the drawing, a part indicated by symbol 60 is an external connector for enabling the connection with the outside. Symbol 25 indicates a temperature sensor that measures an outside temperature. As described later, at the time of empirically determining a heat resistance ratio K, the heat resistance ratio K is corrected using an outside temperature $T5$ measured by the temperature sensor 25. The temperature sensor 25 is disposed adjacently to a region that forms the first region 11 and the second region 12. The measurement of outside air can be also realized by one module and hence, as considered as whole, the deep part thermometer 500 having a small size and light weight can be realized at a low cost.

In this specification, there may be a case where the pair of temperature sensors 21, 22 is referred to as "the pair of first region temperature sensors 21, 22", and the pair of temperature sensors 23, 24 is referred to as "the pair of second region temperature sensors 23, 24" respectively.

"The first region 11" and "the second region 12" are regions where "a first heat flow path 115" and "a second heat flow path 125" that are designed to have a difference between the respective heat resistance values R1, R2 in performing the deep part temperature measurement by a dual-heat flow method are provided respectively (see Fig. 1 (b), Fig. 2 and the like).

External connecting terminals 28 of the respective temperature sensors 21, 22, 23, 24 are connected to the terminals 13c of the board 10 by way of "solder 51". With such a configuration, the respective temperature sensors 21, 22, 23, 24 and the terminals 13c of the board 10 are electrically connected to each other, and the respective sensors are fixed onto the board 10.

The thermal pads 29 of the temperature sensors 23, 24 are connected to the land 13b of the board 10 over the entire surfaces of the respective overlapping regions by way of "solder 51". With such a configuration, the thermal coupling between the thermal pads 29 of the temperature sensors 23, 24 and the land 13b of the board 10 (the nodes as the connecting points of the second heat flow path 125) can be improved and, at the same time, the distances between the thermal pads 29 and the land 13b can be fixed by "solder 51" and hence, a dynamic distance change (estimating a case where only a gap exists without the solder 51) can be suppressed.

In performing soldering, a thickness of "the solder 51" is controlled such that variations fall within a predetermined range so that distances between the thermal pads 29 and the land 13b can be set to a substantially fixed value. Accordingly, a distance between the pair of the second region temperature sensors 23, 24 can be held at a substantially fixed vales and hence, a thermal resistance value R2 of the second heat flow path 125 in manufacturing the deep part temperature measuring probes 1 on a mass production basis can be controlled to a substantially fixed value with favorable reproducibility.

Next, to focus on the second region 12 of the board 10, the second heat flow path 125 is constituted of: the board 10 itself; and the "solder 51" interposed between the thermal pad 29 and the land 13b. The second heat flow path 125 can be constituted by making use of the board 10 itself and hence, it is unnecessary to apply a specific design or a specific technique and, at the same time, it is unnecessary to specifically increase the number of members whereby the probe can be constituted with a simple configuration thus providing an economically advantageous probe.

A second heat flow 120a is formed so as to flow from the rear side to the front side of the deep part temperature measuring probe 1 through the second heat flow path 125 (see symbol 120a schematically indicated in a bold arrow in Fig.

1 (a)).

(4) Configuration of the first heat flow path 115

On the other hand, to focus on the first region 11 of the board 10, a through hole 15 that penetrates between the front surface 10a and the back surface 10b of the board 10 is formed in the board 10 just below the temperature sensors 21, 22 (first region temperature sensors 21, 22). The pair of the first region temperature sensors 21 and 22 is connected to each other through the through hole 15. In such a configuration, "connected" means the pair of the first region temperature sensors 21, 22 is spatially connected to each other, and also means that the first region temperature sensors 21, 22 are connected to each other from a viewpoint of a heat circuit.

Further, in the deep part temperature measuring probe 1 according to the embodiment 1, an air layer 30 is disposed in the through hole 15. Symbol 15a indicates an inner wall of the through hole 15.

The first heat flow path 115 is formed of a gap of the above-mentioned through hole 15 (the air layer 30 in the embodiment 1). In other words, with the provision of the air layer 30 disposed inside the through hole 15 formed in the board 10, a heat register that constitutes the first heat flow path 115 is realized.

Through such a first heat flow path 115, the first heat flow 110a flows from the rear side to the front side of the deep part temperature measuring probe 1 (see symbol 110a schematically indicated by a bold arrow in Fig. 1 (a)).

The first region temperature sensors 21, 22 are each formed of an IC that is a semiconductor. It is preferable that, as viewed in a plan view, the through hole 15 overlaps with the thermal pad 29 of the IC (a back surface of a bare chip in a modification 3 described later) at an overlapping area ratio of 50% or more (see Fig. 1(b)). Further, it is more preferable that the through hole 15 overlap with an entire area of the thermal pad 29.

(5) first heat flow measuring system 110 and second heat flow measuring system 120

The first heat flow path 115 formed in the board in the first region 11 and the pair of first region temperature sensors 21, 22 described above constitute "the first heat flow measuring system 110". In the same manner, the second heat flow path 125 formed in the board in the second region 12 and the pair of second region temperature sensors 23, 24 described above constitute "the second heat flow measuring system 120".

It is also safe to say that the deep part temperature measuring probe 1 according to the embodiment 1 has the following configuration.

That is, the deep part temperature measuring probe 1 includes "the first flow measuring system" that has: the pair of first region temperature sensors 21, 22 that is mounted on the board 10 in the first region 11 of the board 10 in a state where the pair of first region temperature sensors 21, 22 faces each other while sandwiching the board 10 therebetween; and the first heat flow path 115 formed in the board in the first region 11, and measures the first heat flow 110a that flows out from the subject 9. The deep part temperature measuring probe 1 also includes "the second heat flow measuring system 120" that has: the pair of second region temperature sensors 23, 24 that is mounted on the board 10 in the second region 12 of the board 10 in a state where the pair of second region temperature sensors 23, 24 faces each other while sandwiching the board 10 therebetween; and the second heat flow path 125 formed in the board in the second region 12, and measures the second heat flow 120a that flows out from the subject 9. Further, the through hole 15 that penetrates the board 10 between the front surface and the back surface of the board 10 is formed just below the first region temperature sensors 21, 22, and the first heat flow path 115 is formed of the air layer 30 that is disposed in the through hole 15.

2. Configuration of deep part thermometer 500 according to embodiment 1

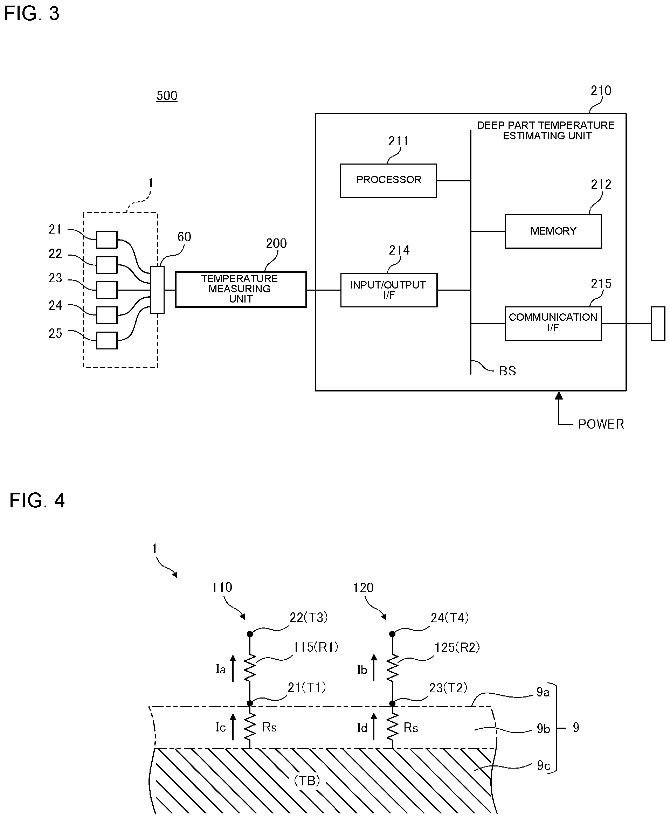

Fig. 3 is a block diagram illustrating one example of the hardware configuration of the deep part thermometer 500 according to the embodiment 1. Symbol 200 indicates a temperature measuring unit.

The deep part thermometer 500 according to the embodiment 1 may be configured to include: the deep part temperature measuring probe 1 according to the embodiment 1 described above; and a deep part temperature estimating unit 210 that estimates a deep part temperature TB using respective temperatures measured by a pair of first region temperature sensors 21, 22 and a pair of second region temperature sensors 23, 24 of the deep part temperature measuring probe 1.

The deep part temperature estimating unit 210 calculates the deep part temperature TB by a dual-heat flow method (by performing an arithmetic operation in accordance with a formula (10) described later, for example), and sets the calculated value as an estimated value.

The deep part temperature estimating unit 210 can be formed of either a dedicated circuit or a general-use circuit. In the case where the deep part temperature estimating unit 210 is formed of the general-use circuit, the deep part temperature estimating unit 210 is realized by an information processing device (not indicated by a symbol) illustrated in Fig. 3, for example.

The information processing device that constitutes the deep part temperature estimating unit 210 includes a processor 211, a memory 212, an input/output interface 214, and a communication interface 215. These parts are connected to a bus BS.

The processor 211 is operated in accordance with a program stored in a memory unit (the memory 212 and a storage not illustrated in the drawing) so as to perform a control of the respective units.

The memory unit (not illustrated in the drawing) includes a non-volatile memory device (ROM or the like). In the memory device, a boot program that is executed by the processor 211 at the time of starting the information processing device, a program that is dependent on a hardware of the information processing device and the like are stored. The storage not illustrated in the drawing is constituted of auxiliary memory devices such as a solid state drive (SDD) and a hard disk drive (HDD). The memory 212 suitably stores data and the like necessary for the processor 211 to perform various controls and processing.

The processor 211 calculates a deep part temperature TB by putting temperatures T1 to T4 and the like into the formula (10) described later and sets the calculated deep part temperature TB as an estimated value. In this case, the deep part temperature estimating unit 210 may be also referred to as a function of the processor that estimates the deep part temperature TB by calculation.

The input/output interface 214 performs inputting and outputting from the input/output devices (particularly, the temperature sensors 21, 22, 23, 24, 25 in this embodiment). The communication interface 215 receives data from other electronic equipment via a network or the like, and transmits the data to the processor 211. Further, the communication interface 215 transmits data that is generated by the processor 211 to the other electronic equipment via the network or the like.

3. Experimental system

In the formula (10) described later that is used at the time of estimating the deep part temperature TB, it is necessary to identify and prepare a value of a heat resistance ratio K between a first heat flow measuring system 110 and a second heat flow measuring system 120. In a case where materials for constituting the first heat flow path 115 and the second heat flow path 125 and physical properties of the first heat flow path 115 and the second heat flow path 125 are already known, it is possible to theoretically obtain the heat resistance ratio K in advance. However, this time, inventors of the present invention have also developed a method where a heat resistance ratio K that conforms to a real state is empirically obtained by a different experimental system in advance and, thereafter, such a heat resistance ratio K is applied to the formula (10). The experimental system is described in detail hereinafter.

(1) Thermal equivalent circuit of deep part temperature measuring probe 1

Fig. 4 is a thermal equivalent circuit diagram of the deep part temperature measuring probe 1 according to the embodiment 1.

In Fig. 4, Ia indicates a heat flow (value) that flows through the first heat flow path 115, and Ib indicates a heat flow (value) that flows through the second heat flow path 125. Ic indicates a heat flow (value) that flows through a heat resistance Rs (heat resistance value) between a subject deep part 9c and the surface of a subject 9a (skin) in the first heat flow measuring system 110, and Id indicates a heat flow (value) that flows through a heat resistance Rs (heat resistance value) between the subject deep part 9c and the surface of the subject 9a (skin) in the second heat flow measuring system 120.

From the thermal equivalent circuit illustrated in Fig. 4, the following formulas are established with respect the first heat flow measuring system 110.

$$Ia = (T_1\text{-}T_3)/R_1 \ldots \quad (1)$$

$$Ic = (TB\text{-}T_1)/Rs \ldots \quad (2)$$

$$Ia = Ic = (T_1\text{-}T_3)/R_1 = (TB\text{-}T_1)/Rs \ldots \quad (3)$$

Formula (4) is established from the formula (3).

$$TB = T_1 + (T_1\text{-}T_3)\times (Rs/R_1)\ldots (4)$$

In the same manner, the following formulas are established with respect the second heat flow measuring system

120.

$$Ib = (T2-T4)/R2 \dots \quad (5)$$

$$Id = (TB-T2)/Rs \dots \quad (6)$$

$$Ib = Id = (T2-T4)/R2 = (TB-T2)/Rs \dots \quad (7)$$

Formula (8) is established from the formula (7).

$$TB = T2 + (T2-T4) \times (Rs/R2) \dots (8)$$

In the above processing, there is a method where a deep part temperature TB is obtained by eliminating Rs or the like using the formula (4) and the formula (8). However, as the result of the experiment, the inventors found that it is difficult to obtain an accurate deep part temperature.

In view of the above, the inventors obtained a deep part temperature TB by another method.

First, from the thermal equivalent circuit illustrated in Fig. 4, a heat resistance ratio K between the first heat flow measuring system 110 and the second heat flow measuring system 210 is defined as follows.

$$K = [(TB-T2)(T1-T3)]/[( (TB-T1)(T2-T4)] \dots (9)$$

Then, the formula (9) can be transformed into the following formula, and the deep part temperature TB can be obtained using this formula.

$$TB = T1 + (T1-T2)(T1-T3)/[K(T2-T4)-(T1-T3)] \dots \quad (10)$$

The inventors performed a provisional experiment based on such an idea, and determined a heat resistance ratio K using the relationship expressed in the formula (9). Then, by using the relationship expressed in the formula (10), a deep part temperature TB was calculated (estimated) based on the heat resistance ratio K determined in the provisional experiment and temperatures (T1, T2, T3, T4) that are measured by the temperature sensors (15, 16, 17, 18).

According to the verification obtained by performing the experiment, it is confirmed that an accurate deep part temperature can be obtained.

That is, it is confirmed that, the deep part temperature estimating unit 210 in the embodiment 1 is configured to estimate a deep part temperature TB in such a manner that, with respect to a heat resistance ratio K between the first heat flow path 115 between the pair of first region temperature sensors 21, 22 and the second heat flow path 125 between the pair of second region temperature sensors 23, 24, the heat resistance ratio K that is preliminarily determined by using the relationship expressed by the formula, that is, K = [(TB-T2)(T1-T3)]/[((TB-T1)(T2-T4)], and the temperatures T1, T2, T3 and T4 that are measured by applying probing to the subject 9 are put into the relationship expressed by the formula, that is, TB = T1 + (T1-T2)(T1-T3)/[K(T2-T4)-(T1-T3)].

In the above-mentioned processing, assume a deep part temperature as TB, a temperature measured by the sensor 21 disposed on a side of the subject 9 out of the pair of first region temperature sensors as T1, a temperature measured by the sensor 22 disposed on the side of the subject 9 out of the pair of second region temperature sensors as T2, a temperature measured by the sensor 23 disposed on a side opposite to the side of the subject 9 out of the pair of first region temperature sensors as T3, and a temperature measured by the sensor 24 disposed on a side opposite to the side of the subject 9 out of the pair of second region temperature sensors as T4.

It may be considered that formulas (2. 6) in the non-patent literature 1 correspond to the formula (10) in this specification. However, it must be noted that, when the inventors of the present invention verified the validity of the formulas (2. 6) in the non-patent literature 1, there is a possibility that formulas (2. 6) in the non-patent literature 1 include errors.

(2) Constitution of experimental system

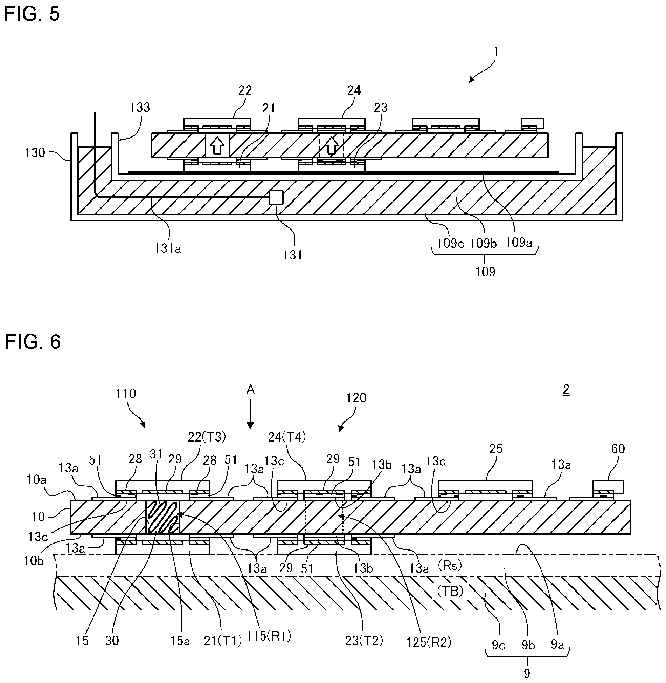

Fig. 5 is a cross-sectional view schematically illustrating "an experimental system" for empirically obtaining a heat resistance ratio K of the deep part temperature measuring probe 1.

As illustrated in Fig. 5, a water bath 130 having a large heat capacity is used in place of the subject deep part 9c, and the water bath 130 is disposed in a constant temperature and humidity bath (not illustrated in the drawing). A temperature (an environment temperature) in the constant temperature and humidity bath is set to a predetermined temperature that falls within a range of from 10°C to 30°C. A water temperature (deep part temperature TB) is set to an approximately fixed value (about 37°C). Symbol 109 indicates a substitute subject, and a surface 109a of the substitute subject is formed of a substitute skin that is formed by simulating a biological skin made of a natural rubber sheet. Symbol 109b indicates a part that corresponds to an intermediate portion of the substitute subject, and symbol 109c indicates a part that corresponds to a deep part of the substitute subject. Symbol 131a indicates a support rod that supports a temperature sensor 131 (a thermistor or the like).

The deep part temperature measuring probe 1 is disposed on an aluminum tub 133, and floats the aluminum tub 133 on water in the water bath 130.

With the use of such an experimental system, it is possible to obtain (i) an actual deep part water temperature (deep part temperature TBr) in water bath 130 by measuring the temperature using the temperature sensor 131. Further, temperatures obtained by sensing using the temperature sensors 21 to 24 of the deep part temperature measuring probe 1 are measured by the temperature measuring unit 200 and are outputted to the deep part temperature estimating unit 210, and the deep part temperature estimating unit 210 performs an arithmetic operation based on such temperatures and hence, it is also possible to obtain (ii) an estimated deep part water temperature (deep part temperature TBp).

(3) Determination of heat resistance ratio K

A heat resistance ratio K can be determined by performing the following experiment (a preliminary experiment) as follows using the formula (9).

(a) By performing the preliminary experiment, an actual deep part water temperature (deep part temperature TBr) is measured and, at the same time, temperatures T1 to T4 are measured by the temperature sensors 21 to 24, and a heat resistance ratio K of the deep part temperature measuring probe 1 (in a thermal equilibrium state in the experimental system illustrated in Fig. 5) is obtained by putting these measured temperatures T1 to T4 into the relationship expressed in the formula (9). Such processing is repeated plural times.
(b) An average value of heat resistance ratios K amounting to plural times corresponding to the experiments performed plural times is obtained.
(c) The measured value of the water temperature (the deep part temperature TBr) and the estimated deep part temperature TBp) calculated by putting the average value of the heat resistance values K and the temperatures T1 to T4 into the relationship expressed in the formula (10) are compared with each other, and it is checked whether or not the difference (an error) between the measured value of the water temperature (deep part temperature Tbr) and the estimated deep part temperature TBp (water temperature) becomes small. In a case where the error is large, the above-mentioned processing (a) to (c) are repeated again, and the heat resistance ratio K with a reduced error is determined.

(4) Estimation of deep part temperature TB

The numerical value of the heat resistance ratio K determined as described above is stored in the memory 212 (see Fig. 3). The temperatures are measured by using the temperature sensors 21 to 24 and the temperature measuring unit 200, and the arithmetic operation is performed by the deep part temperature estimating unit 210 using the formula (10) thus calculating (estimating) the deep part temperature TB.

(5) Assistance using patent application filed prior to this patent application

The content of the patent application (Japanese patent application 2022-53593) filled prior to the present application in which the content of the invention that the inventors of the present invention and et al invented is described (see, for example, Fig. 4, paragraphs [0038] to paragraph [0046] and the like) can be used as a technique for preliminarily empirically determining the heat resistance ratio K of the present invention by incorporating such a technique into the present specification as it is. The detailed description relating to the method of determining the empirical heat resistance ratio K is omitted in this specification.

4. Advantageous effects acquired by deep part temperature measuring probe 1 and deep part thermometer 500 according to embodiment 1

(1) On the board of the deep part temperature measuring probe 1 according to the embodiment 1, the through hole 15 that penetrates the board 10 between the front surface and the back surface of the board 10 is formed just below the first region temperature sensors 21, 22, and the pair of first region temperature sensors 21, 22 are connected to each other through the through hole 15.

That is, in the deep part temperature measuring probe 1, the pair of first region temperature sensors 21, 22 is connected to each other through the through hole 15 formed just below the sensors Accordingly, provided that the board 10 includes only the hole and no particular treatment is applied to the board 10, only the air layer 30 is formed in the through hole 15 and hence, a heat resistance value R1 between the first region temperature sensors 21, 22 can be considerably increased.

Accordingly, even in a case where a heat resistance value of the board 10 in the thickness direction is set small as a whole by making the board 10 that constitutes the probe thin, the difference can be easily made between a heat resistance value R1 of the first heat flow path 115 (between the pair of first region temperature sensors 21, 22) and a heat resistance value R2 of the second heat flow path 125 (between the pair of second region temperature sensors 23, 24).

Accordingly, even in a case where the board 10 that forms the probe is made thin, the deep part temperature measuring probe 1 can measure a temperature (deep part temperature TB) of the subject deep part 9c with high accuracy.

(2) While it is considered that the thermal conductivity of air is 0.0241 [W/(mK)] (at a temperature of 0°C), it is considered that the thermal conductivity of a material used for forming the board 10 (for example, polyimide (PI)) is a value that falls within a range of 0.28 to 0.34 [W/(mK)].

In the deep part temperature measuring probe 1 according to the embodiment 1, the air layer 30 is formed in the through hole 15. Accordingly, to observe the deep part temperature measuring probe 1 from a viewpoint of heat resistance, the air layer 30 can provide an incomparatively large heat resistance value to the deep part temperature measuring probe 1 compared to the members that constitute the board 10 and the like. Accordingly, the heat resistance value R1 of the first heat flow path 115 that is formed of the air layer 15 can be outstandingly increased. Accordingly, the structure for making the difference between the heat resistance value R1 of the first heat flow path 115 and the heat resistance value R2 of the second heat flow path 125 can be realized with the extremely simple structure.

(3) The deep part thermometer 500 according to the embodiment 1 includes: the deep part temperature measuring probe 1 according to the embodiment 1; and the deep part temperature estimating unit 210 that estimates a deep part temperature using the respective temperatures measured by the pair of first region temperature sensors 21, 22 and the pair of second region temperature sensors 23, 24 of the deep part temperature measuring probe 1.

The deep part thermometer 500 includes the deep part temperature measuring probe 1 that can measure a temperature (deep part temperature TB) of the subject deep part 9c with high accuracy even when the thin board 10 is used and hence, it is possible to provide a small-sized and highly accurate deep part thermometer.

(4) The deep part thermometer 500 according to the embodiment 1 is configured to estimate the deep part temperature TB by putting the heat resistance ratio K that is preliminarily determined using the relationship expressed by the above-mentioned formula (9) for obtaining the heat resistance ratio K and the temperatures T1, T2, T3 and T4 that are measured by applying probing to the subject 9 into the relationship expressed by the above-mentioned formula (10).

In an actual operation, it is considered that the heat resistance ratio K is influenced by a heat flux in a diffusion mode besides a linear heat flux. Accordingly, there may be a case where a heat resistance ratio K that is theoretically obtained based on known thermal conductivities of materials that are used for forming the respective heat flow paths differs from an actual accurate heat resistance ratio K. In view of the above, by preliminarily determining the heat resistance ratio K using the relationship expressed in the above-mentioned formula (9) in a different experimental system, it is possible to estimate the deep part temperature TB based on the highly accurate heat resistance ratio K close to that of an actual probe. Accordingly, it is possible to perform the deep part temperature measurement with higher accuracy.

[Embodiment 2] Fig. 6 is a cross-sectional view illustrating a deep part temperature measuring probe 2 according to an embodiment 2. The deep part temperature measuring probe 2 according to the embodiment 2 has basically substantially the same configuration as the deep part temperature measuring probe 1 according to the embodiment 1. However, the deep part temperature measuring probe 2 according to the embodiment 2 differs from the deep part temperature measuring probe 1 according to the embodiment 1 with respect to the manner of forming the configuration of a first heat flow path 115.

As illustrated in Fig. 6, in the deep part temperature measuring probe 2 according to the embodiment 2, a heat insulating sheet 31 is disposed in a through hole 15.

The heat insulating sheet 31 is a sheet having thermal conductivity substantially equal to the thermal conductivity of air. For example, a sheet in which porous particles made of silica gel that contains foamed air (so-called silica aerogel) is impregnated and the like is named as the heat insulating sheet 31. By filling an air layer 30 formed in the through hole 15 with such a heat insulating sheet 31, an entire heat resistance value R1 of the inner space of the through hole 15 becomes substantially equal to the heat resistance value of air. The heat insulating sheet 31 may be filled in the inner space of the through hole 15 in a state that the heat insulating sheet 31 is formed in a suitable stereoscopic shape such as a corrugated shape, a folded state where crests and valleys are formed alternately, or a randomly rounded shape and the like.

By arranging the heat insulating sheet 31 in the through hole 15 that forms the first heat flow path 15, it is possible to allow the heat insulating sheet 31 to obstruct the movement of air in the through hole 15 and hence, the occurrence of convection in the through hole 15 can be suppressed. Accordingly, the transmission and the reception of heat caused by "convection" in the first heat flow path 115 can be suppressed and hence, the direct transmission and reception of heat by the "transfer" via the air layer 30 and the heat insulating sheet 31 is mainly performed. Accordingly, it is possible to realize a more ideal state from a viewpoint of a thermal circuit and hence, the deep part temperature measuring probe 2 can measure a deep part temperature with higher accuracy.

The deep part temperature measuring probe 2 according to the embodiment 2 has basically substantially the same configuration as the deep part temperature measuring probe 1 according to the embodiment 1 except for the manner of constituting the configuration of the first heat flow path 115. Accordingly, the deep part temperature measuring probe 2 according to the embodiment 2 has the corresponding advantageous effects amongst the advantageous effects that the deep part temperature measuring probe 1 according to the embodiment 1 has.

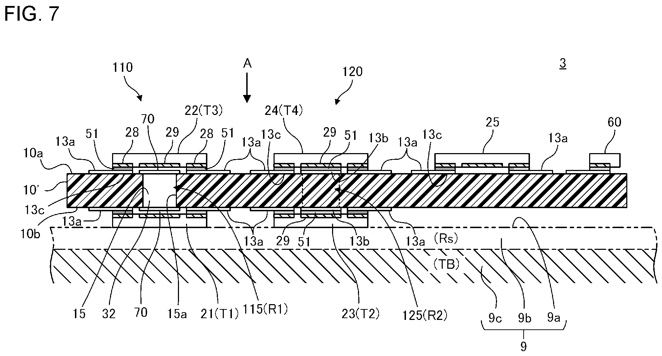

[Embodiment 3] Fig. 7 is a cross-sectional view illustrating a deep part temperature measuring probe 3 according to an embodiment 3. The deep part temperature measuring probe 3 according to the embodiment 3 has basically substantially the same configuration as the deep part temperature measuring probe 1 according to the embodiment 1. However, the deep part temperature measuring probe 3 according to the embodiment 3 differs from the deep part temperature measuring probe 1 according to the embodiment 1 with respect to the configuration of a board 10 and the configuration of a first heat flow path 115.

As illustrated in Fig. 7, in the deep part temperature measuring probe 3 according to the embodiment 3, the board 10 is formed of a glass board, and the inside of a through hole 15 is configured to be brought into a state 32 in vacuum or in a substantially vacuum state.

By bringing the inside of the through hole 15 that forms the first heat flow path 115 into the state 32 in a vacuum or state or in a substantially vacuum state, thermal conductivity of the through hole 15 can be further lowered compared to the case where the through hole 15 is formed of the air layer 30. Accordingly, a heat resistance value R1 between a pair of first region temperature sensors 21, 22 can be further increased.

The deep part temperature measuring probe 3 according to the embodiment 3 has basically substantially the same configuration as the deep part temperature measuring probe 1 according to the embodiment 1 except for the manner of constituting the configuration of the board 10 and the configuration of the first heat flow path 115. Accordingly, the deep part temperature measuring probe 3 according to the embodiment 3 has the corresponding advantageous effects amongst the advantageous effects that the deep part temperature measuring probe 1 according to the embodiment 1 has.

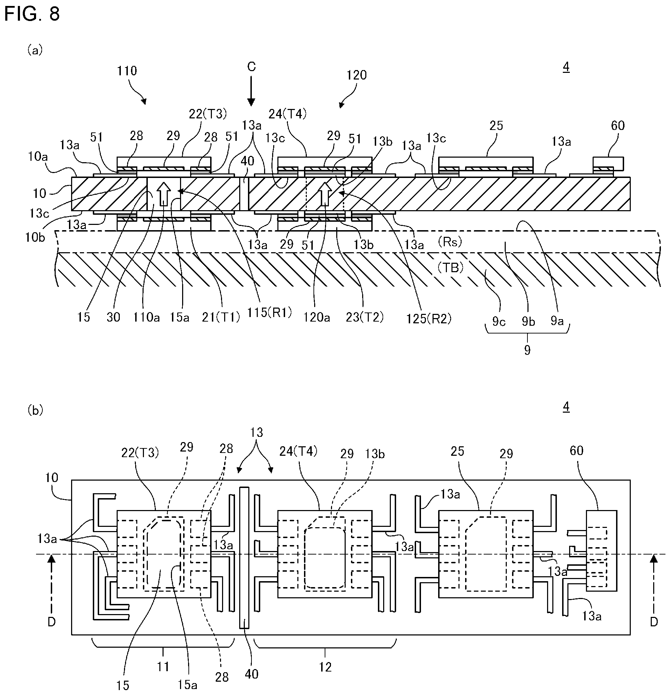

[Embodiment 4] Fig. 8 is a view illustrating a deep part temperature measuring probe 4 according to an embodiment 4. Fig. 8(a) is a cross-sectional view of the deep part temperature measuring probe 4 taken along a line D-D in Fig. 8(b). Fig. 8(b) is a plan view of deep part temperature measuring probe 4 according to the embodiment 4 when the deep part temperature measuring probe 4 is viewed along an arrow C in Fig. 8(a).

The deep part temperature measuring probe 4 according to the embodiment 4 includes basically substantially the same configuration as the deep part temperature measuring probes 1, 2, 3 according to the embodiments 1,2, 3. However, the deep part temperature measuring probe 4 according to the embodiment 4 differs from the deep part temperature measuring probes 1, 2, 3 according to the embodiments 1,2, 3 with respect to a point that the deep part temperature measuring probe 4 according to the embodiment 4 is configured to suppress the thermal interference between a first heat flow path 115 and a second heat flow path 125.

As illustrated in Fig. 8, in the deep part temperature measuring probe 4 according to the embodiment 4, an air layer 40 is formed in the board 10 between a first region 11 and a second region 12. As illustrated in Fig. 8, a through hole that penetrates the board 10 is formed between a front surface 10a and a back surface 10b of the board 10, and the air layer 40 may be formed of air disposed in the through hole. The through hole may be configured as an elongated hole that penetrates a the board 10 in a most portion of a lateral region of the temperature sensor as illustrated in Fig. 8.

The deep part temperature measuring probe 4 according to the embodiment 4 has such a configuration and hence, the air layer 40 that forms a heat insulating body can block the thermal conduction between the first region 11 where the first heat flow path 115 is formed and the second region 12 where the second heat flow path 125 is formed. Accordingly, the thermal interference between the first heat flow path 115 and the second heat flow path 125 can be made small and hence, independency of both heat flow paths can be increased whereby the measurement of a deep part temperature can be performed with higher accuracy.

The deep part temperature measuring probe 4 according to the embodiment 4 has basically substantially the same configuration as the deep part temperature measuring probes 1, 2, 3 according to the embodiments 1, 2, 3 except for the point that the deep part temperature measuring probe 4 according to the embodiment 4 is configured to suppress the thermal interference between the first heat flow path 115 and the second heat flow path 125. Accordingly, the deep part temperature measuring probe 4 according to the embodiment 4 has the corresponding advantageous effects amongst the advantageous effects that the deep part temperature measuring probes 1, 2, 3 according to the embodiments 1, 2, 3 have.

The present invention has been described heretofore based on the embodiments. However, the present invention is not limited to the embodiments described above. The present invention can be carried out in various modes without departing from the gist of the present invention, and the following modifications are conceivable, for example.

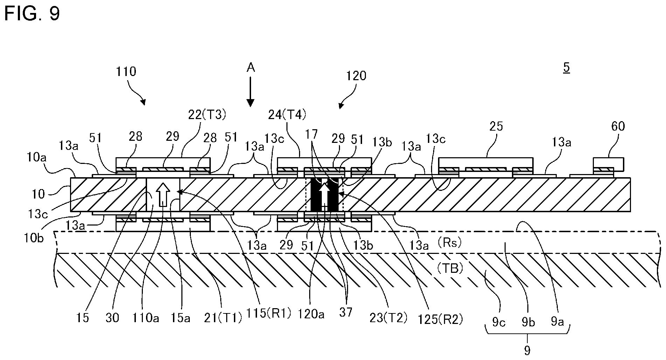

(1) In the respective embodiments, the second heat flow path 125 is formed by making use of the board 10 itself. However, the present invention is not limited to such a configuration. For example, as illustrated in Fig. 9, another through hole 17 that penetrates the board 10 is formed in the board 10 between the front surface 10a and the back surface 10b of the board 10 just below second region temperature sensors 23, 24, metal 37 is embedded into such another through hole 17, and a pair of second region temperature sensors 23, 24 may be connected to each other via the metal 37 (modification 1).

Fig. 9 is a cross-sectional view illustrating a deep part temperature measuring probe 5 according to the modification 1.

As the metal 37, copper can be adopted as an example. It is considered that thermal conductivity of copper is 403 [W/(mK)] (at 0°[C]) and is incomparably large compared to thermal conductivities of members that constitute the board 10 and the like. From a viewpoint of heat resistance, it is possible to allow the metal 37 to have an incomparatively small heat resistance value compared to the members that constitute the board 10 and the like. Accordingly, a heat resistance value R2 of the second heat flow path 125 that is formed of metal 37 can be made considerably small and hence, it is possible to easily secure the difference between the heat resistance value R2 of the second heat flow path 125 and the heat resistance value R1 of the first heat flow path 115.

(2) In the respective embodiments, the description has been made with respect to the case where the solder 51 is connected between thermal pads 29 of the temperature sensors 23, 24 and a land 13b of the wiring pattern 13, as an example. However, the present invention is not limited to such a configuration. For example, the present invention provides the structure that does not have an electrically connection by forming only a gap (an air layer) between the thermal pad 29 and the land 13b of the wiring pattern 13 without interposing "solder 51" therebetween (not illustrated in the drawing). Even with such a structure, thermal coupling can be performed between the pair of second region temperature sensors 23, 24.

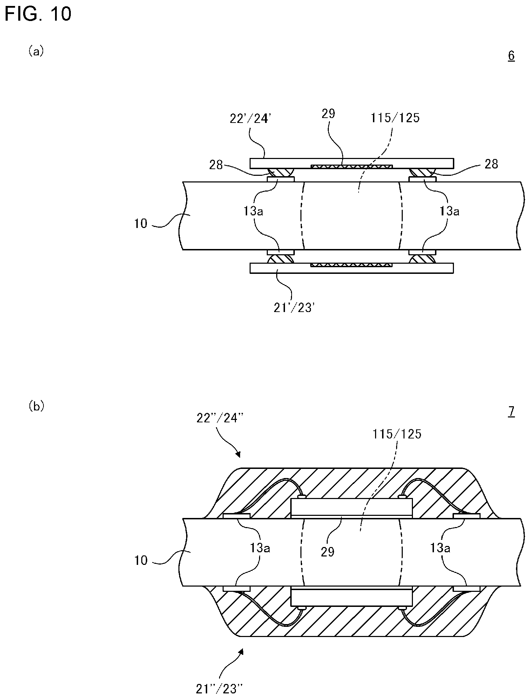

(3) In the respective embodiments, the description has been made with respect to the example where the temperature sensors 21 to 24 are realized by an IC of an SON package. However, the present invention is not limited to such a configuration. For example, as illustrated in Fig. 10(a), the temperature sensors 21 to 24 may be formed of an IC of a wafer level chip size package (WL-CSP) (modification 2). Further, as illustrated in Fig. 10(b), the temperature sensors 21 to 24 may be constituted in a form that bare chips are directly mounted on the board 10 (modification 3). Further, although not illustrated in the drawing, the temperature sensors may each be formed of a temperature sensor such as a thermocouple in place of an IC.

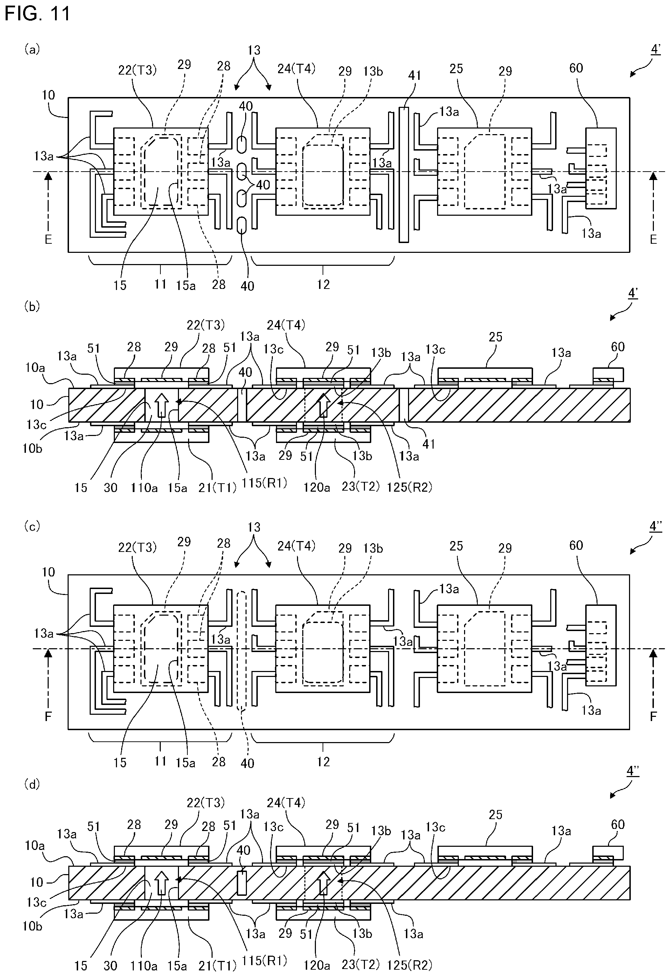

(4) In the deep part temperature measuring probe 4 according to the embodiment 4, the description is made with respect to the case where, to thermally separate the first region 11 and the second region 12 from each other, the "elongated hole shaped" (as viewed in a plan view) penetration hole that penetrates the board 10 between the front surface 10a and the back surface 10b is formed in the board 10. However, the present invention is not limited to such a configuration. For example, as illustrated in Fig. 11(a) and Fig. 11 (b) , the configuration may be adopted where, in place of the elongated hole, spot-like through holes that have a "true circular shape (as viewed in a plan view) and penetrate the board 10 are arranged in a row parallel to each other (modification 4).

Further, for example, as illustrated in Fig. 11(c) and Fig. 11(d), a cavity is formed in the inside of the board 10 in the thickness direction, and an air layer 40 may be formed by filling the cavity with air (modification 5).

Further, for example, as illustrated in Fig. 11, an air layer 41 may be also formed in the board 10 between a region that is constituted of a first region 11 and a second region 12 and a temperature sensor 25. As illustrated in the drawing, the air layer 41 may be formed of an elongated hole that penetrates the most of a region of the board disposed on a side of temperature sensor 25 in a lateral direction.

As viewed from the second region 12, in a plan view of the drawing, the air layer 40 is disposed on the left side, the air layer 41 is disposed on the right side, and an atmosphere 8 (an air layer) is disposed on the upper side and the lower side. In the same manner, as viewed from the first region 11, in the plan view of the drawing, the air layer 40 is disposed on the right side, and the atmosphere 8 (the air layer) is disposed on the left side, the upper side and the lower side. That is, the air layer is disposed around the first region 11 and/or the second region 12.

By adopting the configuration described above, a heat flux between a pair of temperature sensors disposed on a front surface and a back surface of the board 10 is covered/surrounded by the air layer having low thermal conductivity. Accordingly, a heat flow cannot move in a lateral direction as viewed in a plan view and hence, the heat flow is formed of only an ideal heat flow that flows only in a vertical direction and hence, the accuracy of a measurable deep part temperature can be further enhanced. Further, by forming the elongated hole such that a region around the temperature sensor is covered as much as possible or in a continuous manner (around four side, if possible), the more ideal heat flow that flows only in a vertical direction can be obtained and hence, the accuracy of the measurable deep part temperature can be further enhanced. Further, a heat insulating sheet 31 having substantially the same thermal conductivity as air may be disposed in the respective air layers 40, 41.

Fig. 10 is constituted of cross-sectional views illustrating main parts of deep part temperature measuring probes 6, 7 according to the modification 2 and the modification 3. Fig. 11 is constituted of views illustrating deep part temperature measuring probes 4', 4" according to the modifications 4, 5. Fig. 11(a) is a plan view of the deep part temperature measuring probe 4', and Fig. 11(b) is a cross-sectional view of the deep part temperature measuring probe 4' illustrating a cross section taken along a line E-E in Fig. 11(a). Fig. 11(c) is a plan view of the deep part temperature measuring probe 4", and Fig. 11(d) is a cross-sectional view of the deep part temperature measuring probe 4" taken along a line F-F in Fig. 11(c).

Reference signs list

1, 2, 3, 4, 4', 4", 5, 6, 7: deep part temperature measuring probe
8: atmosphere
9: subject
9a: subject surface
9b: subject intermediate part
9c: subject deep part
10: board
10a: front surface (of board)
10b: back surface (of board)
11: first region
12: second region
13: wiring pattern (in broad meaning of the term)
13a: wiring pattern (in narrow meaning of the term)
13b: land
13c: terminal
15: through hole
17: another through hole
21, 22: first region temperature sensor
23, 24: second region temperature sensor
25: temperature sensor
28: external connecting terminal
29: thermal pad
30: air layer
31: heat insulating sheet
32: state in vacuum or in substantially vacuum
37: metal
40, 41: air layer
51: solder
109a: surface of substitute subject
110: first heat flow measuring system
110a: first heat flow
115: first heat flow path
120: second heat flow measuring system
120a: second heat flow
125: second heat flow path
130: water bath
131: temperature sensor
133: aluminum tub
200: temperature measuring unit
210: deep part temperature estimating unit
211: processor
212: memory
214: input/output interface
215: communication interface
500: deep part thermometer

**Claims**

1. A deep part temperature measuring probe that is used at a time of measuring a temperature of a deep part of a subject, the deep part temperature measuring probe comprising:

   a board;
   "a pair of first region temperature sensors" that is a pair of temperature sensors mounted on the board in a first region of the board in a state where the pair of first region temperature sensors face each other while sandwiching the board therebetween; and
   "a pair of second region temperature sensors" that is a pair of temperature sensors mounted on the board in a second region of the board in a state where the pair of second region temperature sensors face each other while sandwiching the board therebetween, wherein
   a through hole that penetrates the board between a front surface and a back surface of the board is formed just below the first region temperature sensor, and the pair of first region temperature sensors are connected to each other through the through hole.

2. The deep part temperature measuring probe according to claim 1, wherein
   an air layer is disposed in an inside of the through hole.

3. The deep part temperature measuring probe according to claim 2, wherein
   a heat insulating sheet is further disposed in the inside of the through hole.

4. The deep part temperature measuring probe according to claim 1, wherein

   the board is formed of a glass board, and
   the inside of the through hole is in a vacuum state or in a substantially vacuum state.

5. The deep part temperature measuring probe according to any one of claims 1 to 4, wherein
   another through hole that penetrates the board is formed in the board from a front surface to a back surface of the board just below the second region temperature sensor, metal is embedded in the another through hole, and the pair of second region temperature sensors are connected to each other via the metal.

6. The deep part temperature measuring probe according to any one of claims 1 to 5, wherein
   an air layer is disposed between the first region and the second region in the board.

7. The deep part temperature measuring probe according to any one of claims 1 to 6, wherein
   an air layer is disposed around the first region and/or the second region in the board.

8. The deep part temperature measuring probe according to any one of claims 1 to 7, wherein
   a temperature sensor that measures outside air is disposed adjacently to regions that form the first region and the second region.

9. A deep part temperature measuring probe that is used at a time of measuring a temperature of a deep part of a subject, the deep part temperature measuring probe comprising:

   a plate-like board;
   a first heat flow measuring system that includes "a pair of first region temperature sensors" that is a pair of temperature sensors mounted on the board in a first region of the board in a state where the pair of first region temperature sensors faces each other while sandwiching the board therebetween, and a first heat flow path formed in the board in the first region, and measures a first heat flow that flows out from a subject; and
   a second heat flow measuring system that includes "a pair of second region temperature sensors" that is a pair of temperature sensors mounted on the board in a second region of the board in a state where the pair of second region temperature sensors faces each other while sandwiching the board therebetween; and a second heat flow path formed in the board in the second region, and measures a second heat flow that flows out from the subject, wherein
   a through hole that penetrates the board between a front surface and a back surface of the board is formed just below the first region temperature sensor, and the first heat flow path is formed of an air layer disposed in the through hole.

10. The deep part temperature measuring probe according to claim 9, wherein
a heat insulating sheet is further disposed in the inside of the through hole.

11. A deep part thermometer comprising:

the deep part temperature measuring probe according to any one of claims 1 to 10; and
a deep part temperature estimating unit that estimates a deep part temperature using respective temperatures measured by the pair of first region temperature sensors and the pair of second region temperature sensors of the deep part temperature measuring probe.

12. The deep part thermometer according to claim 11, wherein

assuming the deep part temperature as TB, and assuming respective temperatures measured by the sensor disposed on a side of the subject out of the pair of first region temperature sensors, the sensor disposed on the side of the subject out of the pair of second region temperature sensors, the sensor disposed on the side opposite to the side of the subject out of the pair of first region temperature sensors, and the sensor disposed on the side opposite to the subject out of the pair of second region temperature sensors as T1, T2, T3 and T4 respectively, the deep part temperature estimating unit is configured to estimate a deep part temperature TB by putting a heat resistance ratio K between the first heat flow path between the pair of first region temperature sensors and the second heat flow path between the pair of second region temperature sensors that is preliminarily determined by using the relationship expressed by the formula $K = [(TB-T2)(T1-T3)]/[(TB-T1)(T2-T4)]$ and the temperatures T1, T2, T3 and T4 that are measured by applying probing to the subject into the relationship expressed by a formula $TB = T1 + (T1-T2)(T1-T3)/[K(T2-T4)-(T1-T3)]$.

FIG. 1

(a)

(b)

FIG. 2

(a)

(b)

FIG. 3

FIG. 4

FIG. 5

FIG. 6

FIG. 7

FIG. 8

(a)

(b)

FIG. 9

FIG. 10

(a)

6

22'/24'  29  115/125

28  28

10  13a  13a

21'/23'

(b)

22"/24"

115/125

10  13a  29  13a

21"/23"

FIG. 11

(a)

(b)

(c)

(d)

| INTERNATIONAL SEARCH REPORT | International application No. |
|---|---|
| | **PCT/JP2023/017069** |

**A. CLASSIFICATION OF SUBJECT MATTER**

**A61B 5/01**(2006.01)i; **G01K 7/42**(2006.01)i; **G01K 13/20**(2021.01)i
FI: A61B5/01 100; G01K7/42 A; G01K13/20 341Z

According to International Patent Classification (IPC) or to both national classification and IPC

**B. FIELDS SEARCHED**

Minimum documentation searched (classification system followed by classification symbols)

A61B5/01; G01K7/42; G01K13/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2016/143518 A1 (OMRON CORPORATION) 15 September 2016 (2016-09-15) entire text, all drawings | 1-12 |
| A | WO 2016/143529 A1 (OMRON CORPORATION) 15 September 2016 (2016-09-15) entire text, all drawings | 1-12 |
| A | JP 2022-521735 A (NEDERLANDSE ORGANISATIE VOOR TOEGEPAST-NATUURWETENSCHAPPELIJK ONDERZOEK TNO) 12 April 2022 (2022-04-12) entire text, all drawings | 1-12 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **04 July 2023** | **18 July 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/017069**

| Patent document cited in search report | | | Publication date (day/month/year) | Patent family member(s) | | | Publication date (day/month/year) |
|---|---|---|---|---|---|---|---|
| WO | 2016/143518 | A1 | 15 September 2016 | US | 2017/0343421 | A1 | |
| | | | | entire text, all drawings | | | |
| WO | 2016/143529 | A1 | 15 September 2016 | US | 2018/0024010 | A1 | |
| | | | | entire text, all drawings | | | |
| JP | 2022-521735 | A | 12 April 2022 | US | 2022/0128413 | A1 | |
| | | | | entire text, all drawings | | | |

Form PCT/ISA/210 (patent family annex) (January 2015)

**REFERENCES CITED IN THE DESCRIPTION**

**Patent documents cited in the description**

- WO 2019167707 A

- JP 2022053593 A

**Non-patent literature cited in the description**

- Development of a non-invasive deep body thermometer and its experimental evaluation. **ISSEI YANAI**. master course thesis of Information Science Research Course of Nara institute of Science and Technology. NAIST Digital Library, 2014